# EUROPEAN PATENT SPECIFICATION

(11) **EP 1 680 386 B1**
(45) Date of publication and mention of the grant of the patent: **07.08.2013**
(21) Application number: 04791685.3
(22) Date of filing: 12.10.2004
(51) Int. Cl.: C07C 27/14, C07C 47/04, C07C 31/04, B01J 23/42, B01J 31/18, B01J 31/22, C07C 29/50, C07C 45/33, C12C 11/02, B01J 31/16

(54) **CONVERSION OF ALKANES HAVING AT MOST 5 CARBONATOMS TO ALCOHOLS AND/OR ALDEHYDES USING A PLATINUM COMPLEX CATALYST**
UMWANDLUNG VON ALKANEN MIT HÖCHSTENS 5 KOHLENSTOFFATOMEN ZU ALKOHOLEN UND/ODER ALDEHYDEN UNTER VERWENDUNG VON PLATINKOMPLEXEN ALS KATALYSATOREN
CONVERSION D'ALCANES PORTANT AU MAXIMUM 5 ATOMES DE CARBONE EN ALCOOLS ET/OU ALDEHYDES A L'AIDE D'UN CATALYSEUR CONSTITUE D'UN COMPLEXE DE PLATINE

(30) Priority: 15.10.2003 ZA 200308023
(43) Date of publication of application: 19.07.2006
(73) Proprietor: Eskom Holdings Limited, 2157 Sunninghill (ZA)
(72) Inventor: LINKOV, Vladimir Mikhailovich, 7130 SOMERSET WEST (ZA); BELYAKOV, Vladimir Nikolaevich, KIEV, 03164 (UA); BELYAKOV, Ludmila Alekseevna, KIEV, 03164 (UA)
(74) Representative: Chalk, Anthony John
(86) International application number: PCT/IB2004/003319
(87) International publication number: WO 2005/037746

(56) References cited:
- EP-A- 0 934 774
- US-A- 4 082 699
- OLAF DEUTSCHMANN ET.AL.: "FORMAL TREATMENT OF CATALYTIC COMBUSTION AND CATALYTIC CONVERSION OF METHANE" CATALYSIS TODAY, vol. 46, 1998, pages 155-163, XP002320964

## Description

THIS INVENTION relates to catalytic conversion, More particularly, the invention relates to a process for the catalytic conversion of alkanes to products selected from the group consisting of alcohols and aldehydes, and to a catalyst for use in the process.

O. Deutschmann et al, "Formal Treatment of Catalytic Combustion and Catalytic Conversion of Methane", Catalysis Today, 46 (1998), 155-163, discuss the catalytic combustion and conversion of methane over platinum foil that is heated resistively. However, there is no suggestion of the use of platinum complexes for this purpose,

US-A-4082699 discloses platinum catalysts prepared by the deposition of colloidal meta-stable platinum metal particles on surface of substrates such as those obtained by the oxidation of complex sulphites with hydrogen peroxide. However, the document makes no mention of the presence of amino, hydroxyl, chloro or imidazolyl ligands in these catalysts,

According to the invention there is provided a gas-phase process for the catalytic conversion of an alkane comprising at most 5 carbon atoms to at least one product selected from the group consisting of alcohols, aldehydes and mixtures thereof, the process comprising bringing a said alkane into contact with an oxidising agent in the presence of a catalyst comprising at least one platinum complex.

The catalyst may be a heterogeneous catalyst, each platinum complex being supported on a catalyst support. The catalyst support may comprise at least one solid metal oxide, the support having a surface to which each platinum complex is chemically bonded. Each metal oxide may be selected from the group consisting of alumina (Al₂O₃), silica (SiO₂), titania (TiO₂), zirconia (ZrO₂) and molybdena (Mo₂O₃), silicon and molybdenum for this purpose being regarded as metals. The catalyst support may comprise a plurality of said metal oxides. In particular, each platinum complex may have two ligands which are chemically bonded to the surface of the metal catalyst support. The catalyst may have, for example, the following structure: 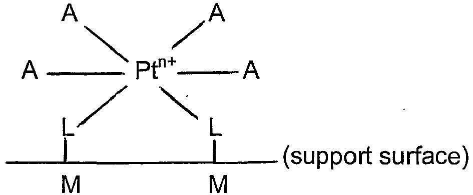 in which:
each L is a ligand selected from amino ligands, hydroxyl ligands and imidazolyl ligands;
each A is a ligand selected from hydroxyl ligands, chloro ligands and amino ligands;
each M is a metal cation of the catalyst support and is selected from the cations of aluminium, silicon, zirconium, titanium and molybdenum; and
n is an integer selected from 4, 2 and 1,
the Pt of the catalyst being a platinum cation having a valence state selected from the valence states (IV), (II) and (I), corresponding to the numerical value of n, said platinum cation forming a central core of the platinum complex, the L ligands and the A ligands surrounding the central core, and the L ligands acting chemically to bond the complex to the surface of the support, under which surface the metal cations M are located.

The catalyst, including the catalyst support, may have a platinum content of 1-6 mg/cm³, preferably 2-5 mg/cm³.

The alkane may be methane, the catalytic conversion being direct catalytic conversion by partial oxidation of the methane to form a product selected from the group consisting of methanol, formaldehyde and mixtures thereof. This is expected to be a particularly useful application of the process of the present invention.

The oxidising agent may be molecular oxygen (O₂), the oxidising agent being contacted in the gas phase with the alkane. Typically the O₂ will be a component of air or a suitable air/oxygen mixture which is contacted in the gas phase with the alkane. It follows that the molecular oxygen may be diluted by molecular nitrogen (N₂).

The process may be carried out at an elevated temperature. Thus the alkane and oxidising agent may be mixed together to form a reaction mixture, the reaction mixture being brought into contact with the catalyst at a reaction temperature which is above ambient temperature. The reaction temperature may be in the range 60-120°C, usually 90-120°C, although any temperature in the range 30-180°C can in principle be employed, if desired.

The pressure at which the process is carried out is not critical. Thus, the reaction mixture may conveniently be brought into contact with the catalyst at ambient atmospheric pressure.

The catalyst may be supported on a solid support contained in a reactor, the reaction mixture being fed into the reactor and into contact with the catalyst at a space velocity of 2 - 20cm³ reaction mixture/cm³ reactor volume/minute. In particular the catalyst may be supported on a solid support contained in a reactor, the support having a porous surface and a porous interior, and the reaction mixture being passed in the reactor over the supported catalyst and through the porous interior of the support.

The support may be in porous sheet or tube form, the support separating a reagent inlet to the reactor from a product outlet from the reactor, the reaction mixture being caused to pass through the porous interior of the support in a direction from the inlet to the outlet.

The catalyst support may in other words be porous and may be in porous sheet form, the porous sheet being flat or optionally formed into a tube so that the support is tubular, the support being fixed in the interior of the reactor, and the reaction mixture being passed through the support and over the catalyst in the reactor. As indicated above the catalyst support may comprise a plurality of said metal oxides as components thereof, being, for example, a bi-component or multi-component mixture to said metal oxides. In this regard it is to be noted, as also indicated above, that the present specification regards silica (Si) and molybdenum (Mo) to be metals. Furthermore, the catalyst support may comprise, in addition to said metal oxides, additional components such as aluminosilicates, zeolites, porous ceramic oxides, fibrous oxides, and additional metal oxides such as bi-component and multi-component metal oxides.

The invention extends to a catalyst comprising a platinum complex supported on a metal oxide support and having the structure: 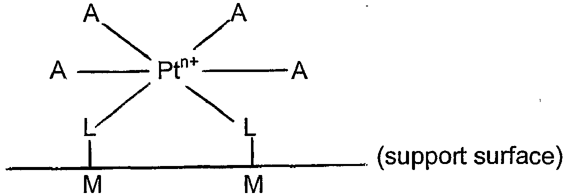 in which:
each L is a ligand selected from amino ligands, hydroxyl ligands and imidazolyl ligands;
each A is a ligand selected from hydroxyl ligands, chloro ligands and amino ligands;
each M is a metal cation of a solid catalyst support comprising at least one metal oxide selected from alumina, silica, zirconia, titania and molybdena, M correspondingly being selected from the cations of aluminium, silicon, zirconium, titanium and molybdenum; and
n is an integer selected from 4, 2 and 1,
the Pt of the catalyst being a platinum cation having a valence state selected from the valence states (IV), (II) and (I) and corresponding to the numerical value of n, said platinum cation forming a central core of the platinum complex, the L ligands and the A ligands surrounding the central core, and the L ligands acting chemically to bond the complex to the surface of the support, under which surface the metal cations M are located.

Preferably,
each ligand L is selected from amino ligands and hydroxyl ligands;
each ligand A is selected from hydroxyl ligands and chloro ligands;
each metal cation M is silicon; and
n has a value of 4,
the platinum cation of the catalyst having a valence state of (IV). As indicated above, the catalyst support may be in porous sheet form, the porosity of the sheet permitting passage of a gas-phase reaction mixture through the sheet from one side of the sheet to another. The porous sheet may be formed into one or more tubes so that the catalyst support is tubular, or it may be in flat sheet form.

The invention extends further to a product, in particular an alcohol or an aldehyde, whenever produced by the process described and defined above.

The catalyst of the present invention typically comprises polyvalent platinum as a component of a poly-ligand complex bonded to a metal oxide surface. Platinum cations can be present in three oxidation states, corresponding respectively to the valence states (IV), (II) and (I). Without being bound by theory and without the invention's being fully understood, it is believed that, during the alkane oxidation process, Pt (IV) is reduced to Pt (II) and possibly also to Pt (I). Simultaneously with alkane oxidation, the oxygen from the oxidizing agent (molecular oxygen optionally as part of air or diluted thereby) present in the reaction mixture re-oxidizes the platinum, thus returning it to the highest oxidation state (IV).

The catalyst of the present invention typically comprises the platinum complex bonded to a metal oxide surface, which acts as a catalyst support, and in this case, two ligands of the platinum complex are usually chemically bonded to surface metal atoms of the support. The structure of one of the more active supported platinum complexes, mentioned above and attached to the surface of a silica support, is as follows:

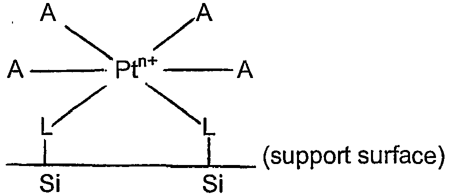

In this complex, as indicated above, L is an amino ligand or a hydroxyl ligand, A is a hydroxyl ligand or a chloro ligand, M is silicon and n is 4. According to the mechanism discussed above, platinum cations in the surface complex are present in the valence state of (IV).

As indicated above, the catalyst of the present invention typically consists of the platinum complex chemically bonded to the surface of the support. Suitable supports include the abovementioned oxides of aluminium, silicon, titanium, zirconium, molybdenum and optionally also the abovementioned other suitable metal oxides and mixtures thereof, such as aluminosilicates, zeolites, porous oxides, fibrous oxides, bi-component mixed oxides and multi-component mixed oxides.

Preferred supports are manufactured from porous metal oxides and are in the form of flat sheets or tubes. Fibrous and globular ceramic materials containing oxides of each of silicon, aluminium and zirconium can be used as starting materials for porous support preparation. The porous structure of the support can be modified by introducing additional ceramic material comprising one of the metal oxides mentioned above into a near-surface layer of the support. Platinum-containing complexes can then be attached to the support surface.

As indicated above, a platinum content in the catalyst may be employed of 1 - 6 mg platinum/cm³ catalyst, preferably 3 - 6 or 3 - 5mg/cm³. The most preferable platinum content is about 3 mg/cm³. It is believed that a content of about 3 mg/cm³ allows for an even distribution of platinum on the catalyst surface and in its porous interior.

The process described in the present application is conveniently carried out in thermally-controlled reactors (reactor vessels) made of stainless steel. The interiors of the reactors constitute reaction zones which are partitioned by flat-sheet and/or tubular porous supported catalyst elements. Reaction mixtures containing selected quantities of selected alkanes and air and/or oxygen at selected temperatures and pressures are passed through the porous supports in the reaction zones and are then removed from the reactions zones. The condensible reaction products (water, alcohols, aldehydes and carbon dioxide) can be investigated chromatographically.

The benefits of using porous catalytic elements in the flat-sheet or tubular form in the reactors are as follows:
(a) Reduction of reaction rate-limiting factors, such as diffusion of the reaction mixture into catalyst particles, which diffusion plays an important role in conventional fixed-bed granular catalytic systems;
(b) Decrease in the contact time between the reaction mixture and the catalyst; and
(c) Reduction of the possibility of complete alkane oxidation to carbon oxides such as carbon monoxide or carbon dioxide, thus increasing the alcohol and aldehyde selectivity of the process.

Under the conditions described above, the time of contact between the catalyst and the reaction mixture is determined by the velocity of gas flow through the ceramic catalytic elements.

The invention will now be described, by way of non-limiting illustrative example, with reference to the following experiments and with reference to the accompanying Tables, for the catalytic conversion, according to the process of the present invention, of methane to methanol and aldehyde, carried out in the gas phase. The reaction temperature was in the range 30 - 180°C, preferably 60 - 120°C, the most preferable temperature range being 90 - 120°C. The oxidizing agents were air and/or molecular oxygen. The methane:oxygen molar ratio in the reaction mixture was in the range 3-5:1. Mixtures with the ratio 5:1 were found to be the most effective reaction mixtures under the laboratory conditions employed.

Based on the experimental results, it is postulated that the reaction mechanism includes reduction of platinum (IV) to platinum (II) and further to platinum (I). In the presence of air or molecular oxygen the catalyst is spontaneously re-oxidized by molecular oxygen (O₂) to the initial valence state of (IV).

The catalysts which were used are set forth in Table 1, which gives details of catalyst supports, catalyst complexes and platinum content. In Table 2 details of the catalytic activities of the catalysts used in the conversion of methane by oxidation are given. The molar ratio of methane (in natural gas) to oxygen in the reaction mixtures was 3-5:1. The reactions were carried out under atmospheric pressure, and at various temperatures and rates of reaction mixture flow through the reactors. Flat-sheet and tubular catalyst supports were used.

The catalysts described in the present application were found to be active at temperatures lower than those required for activity of homogeneous catalysts containing platinum. The main advantages of the catalysts of the present invention were found to be as follows:
(a) Oxidation by means of the catalytic conversion process was found to be possible in the gas phase;
(b) No additional reagents other than alkane (methane or natural gas) and oxygen or air were found to be necessary for performing the catalytic conversion;
(c) Ease of separation of the heterogeneous catalyst from the reaction products was experienced; and
(d) There was an absence of catalyst contamination of the reaction products.

Changing conditions of the catalytic reaction, the reaction mixture flow rate (residence time) and temperature permitted control of the catalyst selectivity towards the formation of either alcohols or aldehydes (methanol and formaldehyde when methane was oxidized) as shown in Table 3. Optimisation of the catalytic process allowed production of either formaldehyde (selectivity 99%) or methanol (selectivity 86%) as main reaction products at methane conversions of 1-10%.

Table 4 shows that the catalysts of the present invention possess catalytic activity towards light alkanes other than methane present in natural gas. Conversion of ethane and butane (n-butane mixed with iso-butane) significantly exceeded that of methane.

The present invention achieves a degree of conversion and methanol selectivity, in the direct oxidation of methane or natural gas by air or molecular oxygen, which is high. The invention permits direct gas-phase oxidation of alkanes to alcohols or aldehydes under mild reaction conditions using molecular oxygen or air. No catalytic promoters of any types, e.g. gaseous chlorine or nitrogen oxides, are required for the process.

**TABLE 1: DESCRIPTION OF CATALYSTS**

| **Catalyst** | **Support** | **Complex of Pt** | **Content of Pt (mg/cm³⁾** |
|---|---|---|---|
| 1 | Fibrous oxides: | L = amino, A = Cl | 1.08 |
| | 47% SiO₂, 40% Al₂O₃, 13% TiO₂ | | |
| 2 | Fibrous oxides: | L = imidazolyl, A = Cl | 1.27 |
| | 47% SiO₂, 46% Al₂O₃, 7% TiO₂ | | |
| 3 | Fibrous oxides: | L = hydroxyl, A = Cl | 1.45 |
| | 50% SiO₂, 42% Al₂O₃, 8% Mo₂O₃ | | |
| 4 | Fibrous oxides: | L = amino, A = amino | 1.45 |
| | 48% SiO₂, 40% Al₂O₃, 12% Mo₂O₃ | | |
| 5 | Fibrous oxides: | L = imidazolyl, A = amino | 2.50 |
| | 53% SiO2, 40% Al₂O₃, 7% TiO₂ | | |
| 6 | Fibrous oxides: | L = hydroxyl, A = amino | 3.00 |
| | 53% SiO₂, 40% Al₂O₃, 7% ZrO₂ | | |
| 7 | Fibrous oxides: | L = amino, A = hydroxyl | 3.20 |
| | 58% SiO₂, 40% Al₂O₃, 2% ZrO₂ | | |
| 8 | Fibrous oxides: | L = imidazolyl, A = hydroxyl | 5.90 |
| | 57% SiO₂, 40% Al₂O₃, 3% ZrO₂ | | |
| 9 | Fibrous oxides: | L = hydroxyl, A = hydroxyl | 6.00 |
| | 59% SiO₂, 40% Al₂O₃, 1 % Mo₂O₃ | | |
| 10 | Fibrous oxides: | L = amino, A = amino | 6.00 |
| | 47% SiO₂, 53% Al₂O₃ | | |
| 11 | Fibrous oxides: | L = imidazolyl, A = amino | 3.20 |
| | 40% SiO₂, 45% Al₂O₃, 15% ZrO₂ | | |
| 12 | Tubular porous ceramic: | L = amino, A = amino | 2.20 |
| | 70% Al₂O₃, 30% ZrO₂ | | |

**TABLE 2: METHANE OXIDATION ACTIVITY OF DIFFERENT CATALYSTS**

| **Catalyst** | **Test Gas** | **T (°C)** | **Flow rate, (cm³/ cm³/ min)** | **Conversion of CH₄ (%)** | **Selectivity (%)** | | |
|---|---|---|---|---|---|---|---|
| | | | | | **CO₂** | **CH₂O** | **CH₃OH** |
| 1 | Natural gas | 90 | 8.0 | 7.33 | 0.0 | 99.1 | 0.9 |
| 2 | Natural gas | 90 | 6.7 | 2.54 | 0.8 | 64.2 | 34.7 |
| 3 | Natural gas | 120 | 6.7 | 3.20 | 2.8 | 94.0 | 3.1 |
| 4 | Natural gas | 90 | 8.0 | 5.09 | 0.0 | 97.0 | 2.9 |
| 5 | Natural gas | 90 | 8.0 | 7.78 | 0.0 | 70.1 | 29.2 |
| 6 | Natural gas | 90 | 8.0 | 4.31 | 0.0 | 97.6 | 3.2 |
| | | 120 | 8.0 | 3.26 | 0.0 | 95.2 | 4.5 |
| 7 | Natural gas | 90 | 8.0 | 2.51 | 0.0 | 12.3 | 87.5 |
| | | 120 | 8.0 | 4.68 | 0.0 | 95.0 | 5.3 |
| 8 | Natural gas | 90 | 8.0 | 0.89 | 0.0 | 36.9 | 63.1 |
| | | 120 | 8.0 | 1.35 | 0.0 | 34.6 | 74.8 |
| 9 | Natural gas | 90 | 8.0 | 1.92 | 0.0 | 34.6 | 65.4 |
| | | | | | | | |
| 10 | CH₄ | 90 | 8.0 | 4.70 | 0.0 | 39.5 | 60.5 |
| 11 | CH₄ | 120 | 8.0 | 5.74 | 0.0 | 91.6 | 8.1 |
| 12 | CH₄ | 90 | 8.0 | 15.4 | 4.2 | 65.7 | 30.0 |

**TABLE 3: FINAL TESTS OF VARIOUS CATALYSTS IN THE CONVERSION BY PARTIAL OXIDATION OF METHANE AND NATURAL GAS**

| **Catalyst** | **Test gas** | **T (°C)** | **Flow rate (cm³/ cm³/ min)** | **Conversion of CH₄ (%)** | **Selectivity (%)** | | |
|---|---|---|---|---|---|---|---|
| | | | | | **CO₂** | **CH₂O** | **CH₃OH** |
| 2 | Natural gas | 60 | 22.3 | 0.49 | 0.0 | 91.2 | 8.7 |
| | | 90 | | 1.54 | 0.0 | 82.4 | 27.3 |
| | | 120 | | 5.05 | 0.0 | 93.0 | 6.2 |
| | | 60 | 11.2 | 0.12 | 0.0 | 87.2 | 22.4 |
| | | 90 | | 2.12 | 0.0 | 54.8 | 45.2 |
| | | 120 | | 3.89 | 0.0 | 88.4 | 11.5 |
| | | 60 | 6.7 | 1.24 | 0.0 | 80.8 | 18.8 |
| | | 90 | | 2.54 | 0.8 | 64.2 | 34.7 |
| | | 120 | | 1.89 | 2.2 | 82.4 | 15.4 |
| | | 60 | 4.4 | 1.45 | 0.5 | 89.1 | 10.2 |
| | | 90 | | 2.34 | 1.8 | 86.4 | 13.5 |
| | | 120 | | 1.50 | 5.6 | 90.0 | 4.3 |
| | | 60 | 11.2 | 1.35 | 0.0 | 63.0 | 36.7 |
| | | 90 | | 1.53 | 0.0 | 47.5 | 52.4 |
| | | 120 | | 2.31 | 0.0 | 87.5 | 12.3 |
| | | 120 | 6.7 | 3.20 | 2.8 | 94.0 | 3.1 |
| | | 120 | 4.4 | 4.23 | 8.5 | 89.4 | 2.1 |
| 10 | CH₄ | 90 | 22.3 | 3.62 | 0.0 | 99.0 | 0.0 |
| | | 120 | | 2.45 | 0.0 | 99.0 | 0.0 |
| | | 90 | 11.2 | 4.01 | 0.0 | 83.6 | 16.4 |
| | | 120 | | 0.21 | 0.0 | 99.0 | 0.0 |
| | | 60 | 8.0 | 0.54 | 0.0 | 86.5 | 15.4 |
| | | 90 | | 4.70 | 0.0 | 39.5 | 60.5 |
| | | 120 | | 4.62 | 0.0 | 99.0 | 0.0 |
| | | 90 | 6.7 | 0.70 | 0.0 | 17.6 | 82.4 |
| | | 120 | | 5.68 | 0.0 | 89.1 | 10.9 |
| | | 90 | 4.4 | 3.08 | 0.0 | 75.9 | 24.1 |
| | | 120 | | 4.62 | 0.0 | 99.0 | 0.0 |
| 11 | CH₄ | 60 | 80 | 1.76 | 0.0 | 99.2 | 0.0 |
| | | 90 | | 0.98 | 0.0 | 99.5 | 0.0 |
| | | 120 | | 5.74 | 0.0 | 91.6 | 8.1 |
| | | 90 | 6.7 | 3.63 | 0.0 | 92.3 | 17.5 |
| | | 120 | | 1.01 | 0.0 | 13.6 | 86.4 |
| | | 90 | 4.4 | 6.73 | 0.0 | 76.3 | 23.7 |
| | | 120 | | 3.23 | 0.0 | 34.6 | 65.4 |
| | | 90 | 2.2 | 10.22 | 0.0 | 85.9 | 14.0 |
| | | 120 | | 6.11 | 0.0 | 72.8 | 23.2 |

**TABLE 4: CONVERSION OF SOME HYDROCARBONS PRESENT IN NATURAL GAS (% VOL)**

| **Content in natural gas (% vol)** | **CH₄** | **C₂H₆** | **C₄H₁₀** |
|---|---|---|---|
| | 90.6 | 3.0 | 1.0 |
| **Catalyst** | **Conversion at 90°C (%)** | | |
| 11(1) | 7.3 | 7.3 | 52.5 |
| 22(3) | 2.3 | 3.3 | 61.3 |
| 18(9) | 1.9 | 3.7 | 25.1 |

## Claims

1. A gas-phase process for the catalytic conversion of an alkane comprising at most 5 carbon atoms to at least one product selected from the group consisting of alcohols, aldehydes and mixtures thereof, the process comprising bringing a said alkane into contact with an oxidising agent in the presence of a catalyst comprising at least one platinum complex.

2. A process as claimed in Claim 1, in which the catalyst is a heterogeneous catalyst, each platinum complex being supported on a catalyst support.

3. A process as claimed in Claim 2, in which the catalyst support comprises at least one solid metal oxide, the support having a surface to which each platinum complex is chemically bonded.

4. A process as claimed in Claim 3, in which each metal oxide is selected from the group consisting of alumina, silica, titania, zirconia, molybdena and mixtures of any two or more thereof, silicon and molybdenum for this purpose being regarded as metals.

5. A process as claimed in Claim 4, in which each catalyst support comprises a plurality of said metal oxides.

6. A process as claimed in any one of Claims 3 - 5 inclusive, in which each platinum complex has two ligands which are chemically bonded to the surface of the catalyst support.

7. A process as claimed in Claim 6, in which the catalyst has the structure: 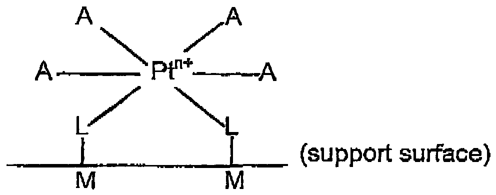 in which:
each L is a ligand selected from amino ligands, hydroxyl ligands and imidazolyl ligands;
each A is a ligand selected from hydroxyl ligands, chloro ligands and amino ligands;
each M is a metal cation of the catalyst support and is selected from the cations of aluminium, silicon, zirconium, titanium and molybdenum; and
n is an integer selected from 4, 2 and 1,
the Pt of the catalyst being a platinum cation having a valence state selected from the valence states (IV), (II) and (I), corresponding to the numerical value of n, said platinum cation forming a central core of the platinum complex, the L ligands and the A ligands surrounding the central core, and the L ligands acting chemically to bond the complex to the surface of the support, under which surface the metal cations M are located.

8. A process as claimed in any one of the preceding claims, in which the catalyst, including the catalyst support, has a platinum content of 1-6 mg/cm³.

9. A process as claimed in Claim 8, in which-the platinum content is 2-5 mg/cm³.

10. A process as claimed in any one of the preceding claims, in which the alkane is methane, the catalytic conversion being direct catalytic conversion by partial oxidation of the methane to form a product selected from the group consisting of methanol, formaldehyde and mixtures thereof.

11. A process as claimed in any one of the preceding claims, in which the oxidising agent is molecular oxygen, the oxidising agent being contacted in the gas phase with the alkane.

12. A process as claimed in Claim 11, in which the molecular oxygen is diluted by molecular nitrogen.

13. A process as claimed in any one of the preceding claims, in which the alkane and oxidising agent are mixed together to form a reaction mixture, the reaction mixture being brought into contact with the catalyst at a reaction temperature which is above ambient temperature.

14. A process as claimed in Claim 13 in which the reaction temperature is in the range 60 - 120°C.

15. A process as claimed in Claim 13 or Claim 14, in which the reaction mixture is brought into contact with the catalyst at ambient atmospheric pressure.

16. A process as claimed in any one of Claims 13-15 inclusive, in which the catalyst is supported on a solid support contained in a reactor, the reaction mixture being fed into the reactor and into contact with the catalyst at a space velocity of 2 - 20cm³ reaction mixture/cm³ reactor volume/minute.

17. A process as claimed in any one of Claims 13-16 inclusive, in which the catalyst is supported on a solid support contained in a reactor, the support having a porous surface and a porous interior, and the reaction mixture being passed in the reactor over the supported catalyst and through the porous interior of the support.

18. A process as claimed in Claim 17, in which the support is in porous sheet or tube form, the support separating a reagent inlet to the reactor from a product outlet from the reactor, the reaction mixture being caused to pass through the porous interior of the support in a direction from the inlet to the outlet.

19. A catalyst for use in a process as claimed in any one of Claims 1-18 inclusive, the catalyst having the structure: 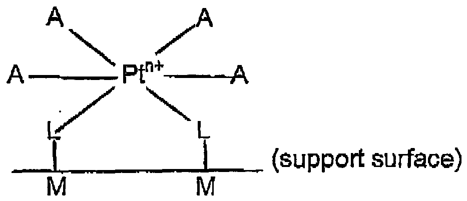 in which:
each L is a ligand selected from amino ligands, hydroxyl ligands and imidazolyl ligands;
each A is a ligand selected from hydroxyl ligands, chloro ligands and amino ligands;
each M is a metal cation of a solid catalyst support comprising at least one metal oxide selected from alumina, silica, zirconia, titania and molybdena, M correspondingly being selected from the cations of aluminium, silicon, zirconium, titanium and molybdenum; and
n is an integer selected from 4, 2 and 1,
the Pt of the catalyst being a platinum cation having a valence state selected from the valence states (IV), (II) and (I) and corresponding to the numerical value of n, said platinum cation forming a central core of the platinum complex, the L ligands and the A ligands surrounding the central core, and the L ligands acting chemically to bond the complex to the surface of the support, under which surface the metal cations M are located.

20. A catalyst as claimed in Claim 19, in which:
each ligand L is selected from amino ligands and hydroxyl ligands;
each ligand A is selected from hydroxyl ligands and chloro ligands;
each metal cation M is silicon; and
n has a value of 4,
the platinum cation of the catalyst having a valence state of (IV).

21. A catalyst as claimed in Claim 19 or Claim 20, the catalyst support being in porous sheet form, the porosity of the sheet permitting passage of a gas-phase reaction mixture through the sheet from one side of the sheet to another.

## Patentansprüche

1. Gasphasenprozess für die katalytische Umsetzung eines Alkans mit höchstens fünf Kohlenstoffatomen in zumindest ein Produkt, das aus der Alkohole, Aldehyde und Mischungen derselben enthaltenden Gruppe ausgewählt ist, wobei der Prozess ein Inkontaktbringen des Alkans mit einem Oxydationsmittel in Gegenwart eines, wenigstens einen Platinkomplex aufweisenden Katalysators beinhaltet.

2. Prozess gemäß Anspruch 1, bei dem der Katalysator ein heterogener Katalysator ist, wobei jeder Platinkomplex von einem Katalysatorträger getragen wird.

3. Prozess gemäß Anspruch 2, bei dem der Katalysatorträger zumindest ein festes Metalloxid aufweist, wobei der Träger eine Oberfläche aufweist, an die jeder Platinkomplex chemisch gebunden ist.

4. Prozess gemäß Anspruch 3, bei dem jedes Metalloxid aus der Aluminiumoxid, Siliziumoxid, Titanoxid, Zirkonoxid, Molybdänoxid und Mischungen zweier oder mehrerer derselben umfassenden Gruppe ausgewählt ist, wobei Silizium und Molybdän für diesen Zweck als Metalle angesehen werden.

5. Prozess gemäß Anspruch 4, bei dem jeder Katalysatorträger eine Vielzahl der Metalloxide beinhaltet.

6. Prozess gemäß einem der Ansprüche 3 - 5, bei dem jeder Platinkomplex zwei Liganden aufweist, die chemisch an die Oberfläche des Katalysatorträgers gebunden sind.

7. Prozess gemäß Anspruch 6, bei dem der Katalysator die Struktur aufweist: 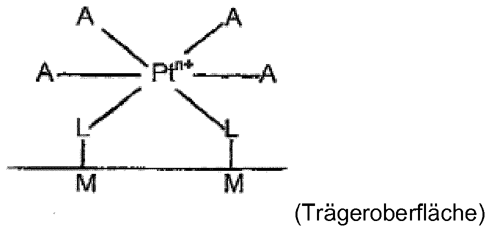 wobei:
jedes L ein Ligand ist, der aus Aminoliganden, Hydroxylliganden und Imidazolylliganden ausgewählt ist;
jedes A ein Ligand ist, der aus Hydroxylliganden, Chlorliganden und Aminoliganden ausgewählt ist;
jedes M ein Metallkation des Katalysatorträgers ist und aus den Kationen Aluminium, Silizium, Zirkon, Titan und Molybdän ausgewählt ist; und
n eine Ganzzahl ist, ausgewählt aus 4, 2 und 1,
das Pt des Katalysators ein Platinkation ist, das einen Valenzzustand aufweist, der aus den Valenzzuständen (IV), (II) und (I) ausgewählt ist, korrespondierend mit dem numerischen Wert von n, wobei das Platinkation einen zentralen Kern des Platinkomplexes bildet, wobei die L-Liganden und die A-Liganden den zentralen Kern umgeben und wobei die L-Liganden chemisch wirken, um den Komplex an die Oberfläche des Trägers zu binden, unter dessen Oberfläche die Metallkationen M angeordnet sind.

8. Prozess gemäß einem der vorhergehenden Ansprüche, in welchem der Katalysator, einschließlich des Katalysatorträgers, einen Platingehalt von 1-6 mg/cm³ aufweist.

9. Prozess gemäß Anspruch 8, in welchem der Platingehalt 2-5 mg/cm³ ist.

10. Prozess gemäß einem der vorhergehenden Ansprüche, bei welchem das Alkan Methan ist, wobei die katalytische Umwandlung eine direkte katalytische Umwandlung durch partielle Oxidation des Methans ist, um ein Produkt auszubilden, das aus der Methanol, Formaldehyd und Mischungen derselben bestehenden Gruppe ausgewählt ist.

11. Prozess gemäß einem der vorhergehenden Ansprüche, bei dem das Oxidationsmittel molekularer Sauerstoff ist, wobei das Oxidationsmittel in der Gasphase mit dem Alkan in Kontakt gebracht wird.

12. Prozess gemäß Anspruch 11, bei dem der molekulare Sauerstoff durch molekularen Stickstoff verdünnt ist.

13. Prozess gemäß einem der vorhergehenden Ansprüche, bei dem das Alkan und das Oxidationsmittel zusammengemischt werden, um eine Reaktionsmischung zu bilden, wobei die Reaktionsmischung mit dem Katalysator bei einer Reaktionstemperatur in Kontakt gebracht wird, die oberhalb der Umgebungstemperatur liegt.

14. Prozess gemäß Anspruch 13, bei dem die Reaktionstemperatur im Bereich von 60-120°C liegt.

15. Prozess gemäß Anspruch 13 oder Anspruch 14, bei dem die Reaktionsmischung mit dem Katalysator bei umgebenden Atmosphärendruck in Kontakt gebracht wird.

16. Prozess gemäß einem der Ansprüche 13 - 15, bei dem der Katalysator von einem in einem Reaktor enthaltenden festen Träger getragen wird, wobei die Reaktionsmischung in den Reaktor hineingegeben wird und mit dem Katalysator bei einer Raumgeschwindigkeit von 2-20 cm³ Reaktionsmischung / cm³ Reaktorvolumen / Minute in Kontakt gebracht wird.

17. Prozess gemäß einem der Ansprüche 13 - 16, bei dem der Katalysator von einem, in einem Reaktor enthaltenden, festen Träger getragen wird, wobei der Träger eine poröse Oberfläche und ein poröses Inneres aufweist, und die Reaktionsmischung über den getragenen Katalysator und durch das poröse Innere des Trägers in den Reaktor geleitet wird.

18. Prozess gemäß Anspruch 17, bei dem der Träger in poröser Platten- oder Röhrenform ausgebildet ist, wobei der Träger einen Reagenzeinlass zum Reaktor von einem Produktauslass vom Reaktor separiert, wobei die Reaktionsmischung zum Durchtreten des porösen Inneren des Trägers in einer Richtung vom Einlass zum Auslass veranlasst wird.

19. Katalysator zur Verwendung in einem Prozess gemäß einem der Ansprüche 1 - 18, wobei der Katalysator die Struktur aufweist: 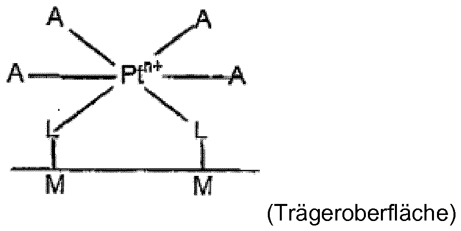 wobei:
jedes L ein Ligand ist, der aus Aminoliganden, Hydroxylliganden und Imidazolylliganden ausgewählt ist;
jedes A ein Ligand ist, der aus Hydroxylliganden, Chlorliganden und Aminoliganden ausgewählt ist;
jedes M ein Metallkation eines festen Katalysatorträgers ist, der zumindest ein Metalloxid aufweist, das aus Aluminiumoxid, Siliziumoxid, Zirkonoxid, Titanoxid und Molybdänoxid ausgewählt ist, wobei M entsprechend aus den Kationen Aluminium, Silizium, Zirkon, Titan und Molybdän ausgewählt ist; und
n eine Ganzzahl ist ausgewählt aus 4, 2 und 1,
das Pt des Katalysators ein Platinkation ist, das einen Valenzzustand aufweist, der aus den Valenzzuständen (IV), (II) und (I) ausgewählt ist und mit dem numerischen Wert von n korrespondiert, wobei das Platinkation einen zentralen Kern des Platinkomplexes bildet, wobei die L-Liganden und die A-Liganden den zentralen Kern umgeben und wobei die L-Liganden chemisch wirken, um den Komplex an die Oberfläche des Trägers zu binden, unter dessen Oberfläche die Metallkationen M angeordnet sind.

20. Katalysator gemäß Anspruch 19, bei dem:
jeder Ligand L aus Aminoliganden und Hydroxlliganden ausgewählt ist;
jeder Ligand A aus Hydroxylliganden und Chlorliganden ausgewählt ist;
jedes Metallkation M Silizium ist; und
n den Wert 4 hat,
wobei das Platinkation des Katalysators einen Valenzzustand von (IV) hat.

21. Katalysator gemäß Anspruch 19 oder Anspruch 20, wobei der Katalysatorträger in einer porösen Plattenform vorliegt, wobei die Porosität der Platte einen Durchgang einer Gasphasenreaktionsmischung durch die Platte von einer Seite der Platte zur anderen erlaubt.

## Revendications

1. Procédé en phase gazeuse pour la conversion catalytique d'un alcane comprenant au plus 5 atomes de carbone en au moins un produit sélectionné dans le groupe constitué par les alcools, les aldéhydes et les mélanges de ceux-ci, le procédé comprenant la mise dudit alcane en contact avec un agent oxydant en présence d'un catalyseur comprenant au moins un complexe de platine.

2. Procédé selon la revendication 1, dans lequel le catalyseur est un catalyseur hétérogène, chaque complexe de platine étant supporté sur un support de catalyseur.

3. Procédé selon la revendication 2, dans lequel le support de catalyseur comprend au moins un oxyde de métal solide, le support ayant une surface à laquelle chaque complexe de platine est chimiquement lié.

4. Procédé selon la revendication 3, dans lequel chaque oxyde de métal est sélectionné dans le groupe constitué par l'alumine, la silice, le dioxyde de titane, l'oxyde de zirconium, l'oxyde de molybdène et les mélanges de deux d'entre eux, ou plus, le silicium et le molybdène à cet effet étant considérés comme des métaux.

5. Procédé selon la revendication 4, dans lequel chaque support de catalyseur comprend une pluralité desdits oxydes de métaux.

6. Procédé selon l'une quelconque des revendications 3 à 5 incluse, dans lequel chaque complexe de platine a deux ligands qui sont liés chimiquement à la surface du support de catalyseur.

7. Procédé selon la revendication 6, dans lequel le catalyseur a la structure suivante : 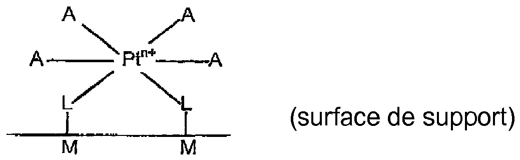 dans laquelle :
chaque L est un ligand sélectionné parmi les ligands amino, les ligands hydroxyle et les ligands imidazolyle ;
chaque A est un ligand sélectionné parmi les ligands hydroxyle, les ligands chloro et les ligands amino ;
chaque M est un cation métallique du support de catalyseur et est sélectionné parmi les cations d'aluminium, de silicium, de zirconium, de titane et de molybdène ; et
n est un entier sélectionné parmi 4, 2 et 1,
le Pt du catalyseur étant un cation de platine ayant un état de valence sélectionné parmi les états de valence (IV), (II) et (I), correspondant à la valeur numérique de n, ledit cation de platine formant un noyau central du complexe de platine, les ligands L et les ligands A entourant le noyau central, et les ligands L exerçant une action chimique pour lier le complexe à la surface du support, surface sous laquelle sont situés les cations métalliques M.

8. Procédé selon l'une quelconque des revendications précédentes, dans lequel le catalyseur, dont le support de catalyseur, a une teneur en platine de 1 à 6 mg/cm³.

9. Procédé selon la revendication 8, dans lequel la teneur en platine est de 2 à 5 mg/cm³.

10. Procédé selon l'une quelconque des revendications précédentes, dans lequel l'alcane est le méthane, la conversion catalytique étant une conversion catalytique directe par oxydation partielle du méthane pour former un produit sélectionné dans le groupe constitué par le méthanol, le formaldéhyde et les mélanges de ceux-ci.

11. Procédé selon l'une quelconque des revendications précédentes, dans lequel l'agent oxydant est l'oxygène moléculaire, l'agent oxydant étant mis en contact avec l'alcane dans la phase gazeuse.

12. Procédé selon la revendication 11, dans lequel l'oxygène moléculaire est dilué par de l'azote moléculaire.

13. Procédé selon l'une quelconque des revendications précédentes, dans lequel l'alcane et l'agent oxydant sont mélangés ensemble pour former un mélange réactionnel, le mélange réactionnel étant mis en contact avec le catalyseur à une température de réaction qui est supérieure à la température ambiante.

14. Procédé selon la revendication 13, dans lequel la température de réaction se situe dans la plage de 60 à 120°C.

15. Procédé selon la revendication 13 ou la revendication 14, dans lequel le mélange réactionnel est mis en contact avec le catalyseur à la pression atmosphérique ambiante.

16. Procédé selon l'une quelconque des revendications 13 à 15 incluse, dans lequel le catalyseur est supporté sur un support solide contenu dans un réacteur, le mélange réactionnel étant amené dans le réacteur et mis en contact avec le catalyseur à une vitesse spatiale de 2 à 20 cm³ de mélange réactionnel/cm³ de volume de réacteur/minute.

17. Procédé selon l'une quelconque des revendications 13 à 16 incluse, dans lequel le catalyseur est supporté sur un support solide contenu dans un réacteur, le support ayant une surface poreuse et un intérieur poreux, et le mélange réactionnel étant passé dans le réacteur sur le catalyseur supporté et à travers l'intérieur poreux du support.

18. Procédé selon la revendication 17, dans lequel le support a la forme d'une feuille poreuse ou d'un tube, le support créant une séparation entre une entrée de réactif dans le réacteur et une sortie de produit hors du réacteur, le mélange réactionnel étant amené à passer à travers l'intérieur poreux du support dans une direction allant de l'entrée à la sortie.

19. Catalyseur destiné à être utilisé dans un procédé selon l'une quelconque des revendications 1 à 18 incluse, le catalyseur ayant la structure suivante : 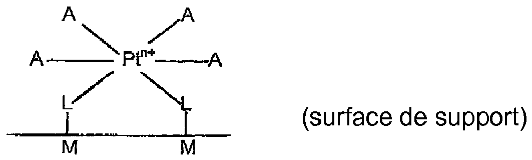 dans laquelle :
chaque L est un ligand sélectionné parmi les ligands amino, les ligands hydroxyle et les ligands imidazolyle ;
chaque A est un ligand sélectionné parmi les ligands hydroxyle, les ligands chloro et les ligands amino ;
chaque M est un cation métallique d'un support de catalyseur solide comprenant au moins un oxyde de métal sélectionné parmi l'alumine, la silice, l'oxyde de zirconium, le dioxyde de titane et l'oxyde de molybdène, M étant en conséquence sélectionné parmi les cations d'aluminium, de silicium, de zirconium, de titane et de molybdène ; et
n est un entier sélectionné parmi 4, 2 et 1,
le Pt du catalyseur étant un cation de platine ayant un état de valence sélectionné parmi les états de valence (IV), (II) et (I) et correspondant à la valeur numérique de n, ledit cation de platine formant un noyau central du complexe de platine, les ligands L et les ligands A entourant le noyau central, et les ligands L exerçant une action chimique pour lier le complexe à la surface du support, surface sous laquelle sont situés les cations métalliques M.

20. Catalyseur selon la revendication 19, dans lequel :
chaque ligand L est sélectionné parmi les ligands amino et les ligands hydroxyle ;
chaque ligand A est sélectionné parmi les ligands hydroxyle et les ligands chloro ;
chaque cation métallique M est un cation de silicium ; et
n a une valeur de 4,
le cation de platine du catalyseur ayant un état de valence de (IV).

21. Catalyseur selon la revendication 19 ou la revendication 20, le support de catalyseur ayant la forme d'une feuille poreuse, la porosité de la feuille permettant le passage d'un mélange réactionnel en phase gazeuse à travers la feuille d'un côté à l'autre.
